Europäisches Patentamt

European Patent Office

Office européen des brevets

(11) Veröffentlichungsnummer: **0 301 359**
**A1**

(12) **EUROPÄISCHE PATENTANMELDUNG**

(21) Anmeldenummer: 88111544.8

(22) Anmeldetag: 18.07.88

(51) Int. Cl.⁴ **A61B 6/08** , **A61B 5/05** , **A61B 5/04**

(30) Priorität: 30.07.87 DE 3725325

(43) Veröffentlichungstag der Anmeldung:
01.02.89 Patentblatt 89/05

(84) Benannte Vertragsstaaten:
**DE FR IT NL**

(71) Anmelder: **Siemens Aktiengesellschaft Berlin und München**
**Wittelsbacherplatz 2**
**D-8000 München 2(DE)**

(72) Erfinder: **Schneider, Siegfried, Dr.**
**Kulmbacher Strasse 33**
**D-8520 Erlangen(DE)**
Erfinder: **Abraham-Fuchs, Klaus, Dipl.-Phys.**
**Graslitzer Strasse 17**
**D-8520 Erlangen(DE)**

(54) **Vorrichtung zur geometrischen Zuordnung von Informationen, die von einem Gegenstand in zwei Untersuchungsgeräten gewonnen sind.**

(57) Nach einer ersten Ausführungsform enthält die Vorrichtung eine Beißplatte (2), deren Kontur dem Gebiß des Patienten (8) entspricht, und einen starren Träger (4), der mit der Beißplatte (2) verbindbar oder verbunden ist und der Markierungen (6, 6') zur Lokalisierung in einem Schnittbildverfahren (das von dem einen Untersuchungsgerät durchgeführt wird) trägt. Nach einer zweiten Ausführungsform enthält die Vorrichtung einen starren Ring (30), der um den Patienten (28) geschlungen wird, eine Anzahl in der Länge veränderbarer Zeiger (34, 35, 36), die jeweils mit einer Skala (44, 45, 46) versehen sind, und einen mit einem Ring (30) verbundenen Körper (62), der für ein Schnittbildverfahren erkennbar geformt oder mit Markierungen (64) versehen ist. Beide Ausführungen werden in dem anderen Untersuchungsgerät, und zwar einem Untersuchungsgerät für biomagnetische Signale, eingesetzt.

FIG 1

## Vorrichtung zur geometrischen Zuordnung von Informationen, die von einem Gegenstand in zwei Untersuchungsgeräten gewonnen sind

Die Erfindung bezieht sich auf eine Vorrichtung zur Zuordnung von geometrischen Informationen über einen Untersuchungsgegenstand, die in einem Schnittbildgerät gewonnen werden, zu Meßpunkten, die von demselben Untersuchungsgegenstand in einer Meßeinrichtung für biomagnetische Signale gewonnen werden.

Die Messung biomagnetischer Signale gewinnt für die medizinische Diagnostik immer größere Bedeutung (vgl. Zeitschrift "Bild der Wissenschaft", Heft 8, 1986, Seiten 76-83). Mit Hilfe von sog. SQUID-Systemen lassen sich äußerst schwache biomagnetische Signale meßtechnisch erfassen, z.B. die der sog. evozierten Magnetfelder des menschlichen Gehirns, die in der Größenordnung von nur $10^{-14}$ T liegen. Der Proband ist bei einer solchen Messung in einem magnetisch abgeschirmten Meßraum untergebracht, und sein Kopf wird mit dem SQUID-System oder -Magnetometer berührungslos abgetastet. Die mit Hilfe der SQUID-Technologie gewonnenen Daten werden sodann mit Hilfe eines Computers unter Zugrundelegung eines vorgegebenen mathematischen Modells ausgewertet. Dabei werden weitere Patientendaten benötigt, insbesondere über die Geometrie, beispielsweise über die Struktur, Größe und Ausdehnung des Gehirns. Diese weiteren Patientendaten können durch weitere Untersuchungen, insbesondere Ultraschall-, Computer-Tomographie (CT)- oder magnetische Resonanz (MR)-Aufnahmen, bereitgestellt werden. Hierbei stellt sich das Problem der Koordination der biomagnetischen Meßergebnisse mit den Ergebnissen der anderen bildgebenden Verfahren, also z.B. der betreffenden Ultraschall-, CT- oder MR-Technologie.

Das angesprochene Problem der Koordination oder Zuordnung läßt sich am Beispiel einer Magneto-Enzephalographie-Messung (MEG) und eines Kernspin-Tomographie-Bildes (MR) noch verdeutlichen, wenn es in Teilprobleme aufgelöst wird:

a) Bestimmung der Lage und Orientierung des Kopfes des Patienten relativ zum flächenhaften Detektor-Array bei der MEG-Messung;

b) Fixierung des Kopfes während der MEG-Messung; und

c) Bestimmung der Lage und Orientierung des Kopfes im MR-Bild relativ zur Lage und Orientierung des Kopfes bei der MEG-Messung, oder umgekehrt.

Erst wenn die angesprochenen Teilprobleme a) bis c) zufriedenstellend gelöst sind, kann jedem der mit dem SQUID-Gradiometer ermittelten Meßpunkte des Kopfes ein Punkt im MR-Bild eindeutig zugeordnet werden. Allgemeiner formuliert lautet die Fragestellung: Welche Zuordnung existiert zwischen den einzelnen Meßpunkten der MEG-Messung und der Kontur des Gehirns, die in einem Schnittbildverfahren ermittelt und in einem Schnittbild dargestellt wird? Noch allgemeiner formuliert lautet die Fragestellung: Welche geometrische Zuordnung von Informationen über einen Untersuchungsgegenstand, die in einem Schnittbildgerät gewonnen wurden, existiert zu Meßpunkten, die von demselben Untersuchungsgegenstand in einer Meßeinrichtung für biomagnetische Signale gewonnen wurden?

Der Erfindung liegt die Aufgabe zugrunde, eine Vorrichtung anzugeben, die die angesprochene Fragestellung der Zuordnung zu beantworten hilft.

Die oben unter a) angesprochene Teilaufgabe allein für sich ist nach einer Broschüre "Probe Position Indicator" der Fa. Biomagnetic Technologies, Inc., San Diego, Kalifornien, USA, vom Juli 1986 wie folgt gelöst: Kleine Spulen werden an definierten Punkten des Kopfes (z.B. Nasion, Inion, Ohrkanal) befestigt. Das Magnetfeld eines die Spulen durchfließenden Stroms wird gemessen, und daraus wird als inverse Lösung der Maxwell-Gleichungen die Position der Spulen und somit der definierten Punkte am Kopf errechnet. Eine Koordination zwischen zwei Meßeinrichtungen wird durch die bekannte Lösung nicht bewirkt.

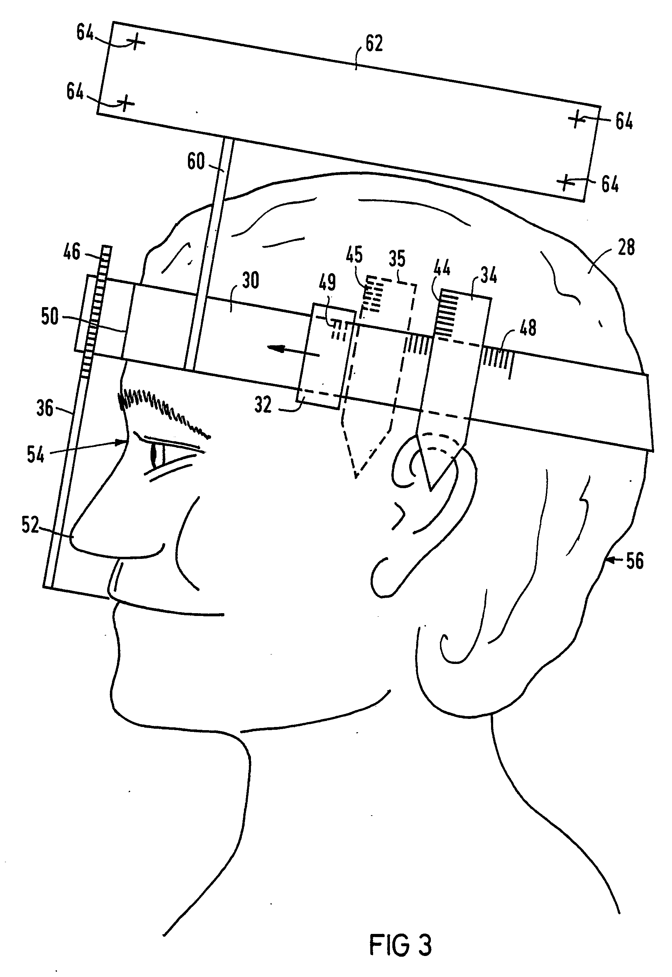

Das unter Punkt b) angesprochene Teilproblem der Fixierung des Kopfes während der MEG-Messung kann nach der Dissertation "The inverse problem in EEG and MEG with application to visual evoked responses" von Cornelis Johannes Stok, Rijksuniversiteit Leiden, Niederlande, 1986, insbes. Seite 46 und Fig. 3.4, durch ein Mundstück oder eine Beißplatte gelöst werden. Diese Beißplatte dient jedoch nur dazu, den Patienten oder den Probanden in einer vorgegebenen Position in bezug auf das SQUID-Gradiometer zu halten. Eine Koordination wird also auch hier nicht herbeigeführt.

Die genannte Aufgabe wird nach einer ersten grundlegenden Ausführungsform erfindungsgemäß gelöst durch

a) eine Beißplatte, deren Kontur dem Gebiß des Patienten entspricht, und

b) einen starren Träger, der mit der Beißplatte verbindbar oder verbunden und der mit Markierungen versehen ist.

Der starre Träger kann prinzipiell einen beliebigen Aufbau haben. Beispielsweise kann er als eine ebene Platte ausgebildet sein. Diese kann beispielsweise aus Kunststoff bestehen. Die ebene Kunststoffplatte ist jedoch nur eine einfache Realisierungsmöglichkeit. Viele andere Formen sind denkbar, beispielsweise eine abgewinkelte Platte. Auch ist es möglich, eine starre Kopplung der Beißplatte mit einem halbkreisförmigen Bügel vorzusehen, der jeweils an den beiden Ohrkanälen des Patienten endet. Durch einen solchen Bügel kann eine Gewichtsentlastung am Kopf des Patienten bewirkt werden.

Bei der Messung wird mit einer solchen Vorrichtung so vorgegangen, daß der Patient auf die Beißplatte beißt und sich der Schnittbilduntersuchung unterzieht. Danach wird die Beißplatte mit der Meßapparatur für die biomagnetischen Signale fest verbunden; der Patient beißt wieder auf die Beißplatte, und eine entsprechende biomagnetische Messung wird in dieser Position durchgeführt. Die in beiden Prozeduren erhaltenen Daten oder Informationen sind fest miteinander korreliert.

Nach einer zweiten grundlegenden Ausführungsform wird die genannte Aufgabe erfindungsgemäß gelöst durch

a) einen starren Ring, der um den Patienten schlingbar ist,

b) eine Anzahl in der Länge veränderbarer Zeiger, die jeweils mit einer Skala versehen und am Ring angebracht sind, und

c) einen mit dem Ring verbundenen Körper, der für ein vorgegebenes Diagnoseverfahren erkennbar geformt oder markiert ist derart, daß seine Lage und Orientierung im Raum definiert sind.

Bei der Messung mit dieser Vorrichtung wird entsprechend obigen Prozeduren vorgegangen.

Der genannte Körper kann entweder eine eckige Platte, ein Kreuz oder eine Kreisscheibe sein.

Während bei der erstgenannten grundlegenden Ausführungsform nur das Gebiß des Patienten als Kopf-Fixpunkt dient, ist bei der zweitgenannten grundlegenden Ausführungsform mehr als ein Fixpunkt, beispielsweise am Kopf des Patienten, erforderlich; zu diesem wird bei der Messung die Vorrichtung über die genannten veränderbaren Zeiger in eine definierte Position gebracht.

Weitere vorteilhafte Ausgestaltungen sind in den Unteransprüchen gekennzeichnet.

Ausführungsbeispiele der Erfindung werden im folgenden anhand von drei Figuren näher erläutert. Es zeigen:

Fig. 1 eine Koordinations-Vorrichtung mit Beißplatte und starrem Träger in Form einer (abgewinkelten) Kunststoff-Platte;

Fig. 2 die geometrischen Verhältnisse der Kunststoff-Platte in Bezug auf Bauteile einer Meßeinrichtung für biomagnetische Signale; und

Fig. 3 eine Koordinations-Vorrichtung mit einem starren Ring und einer Anzahl längenveränderlicher Zeiger.

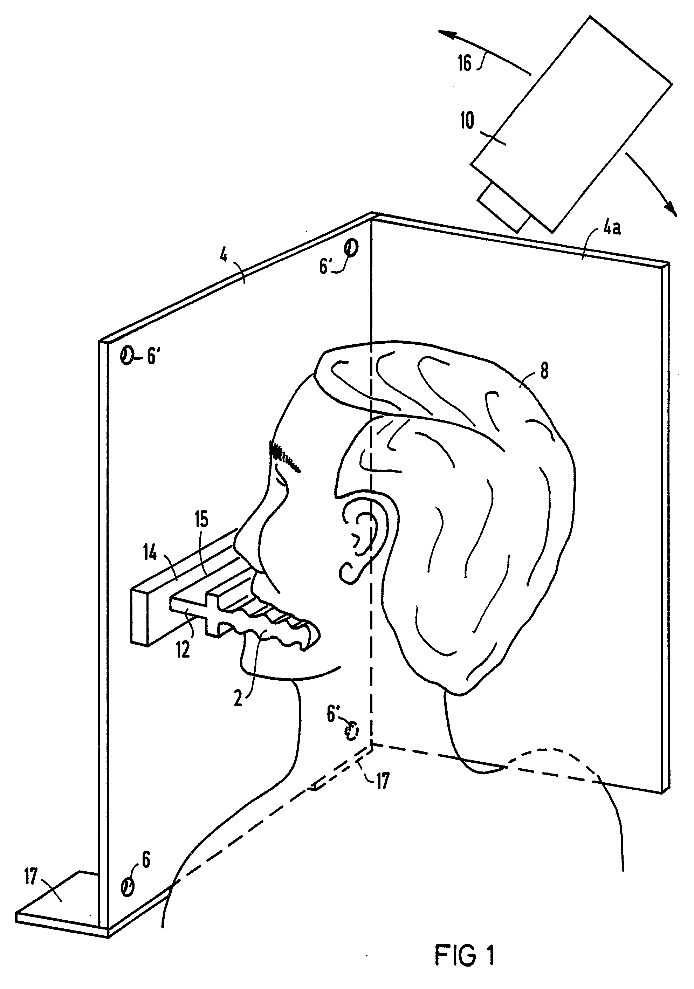

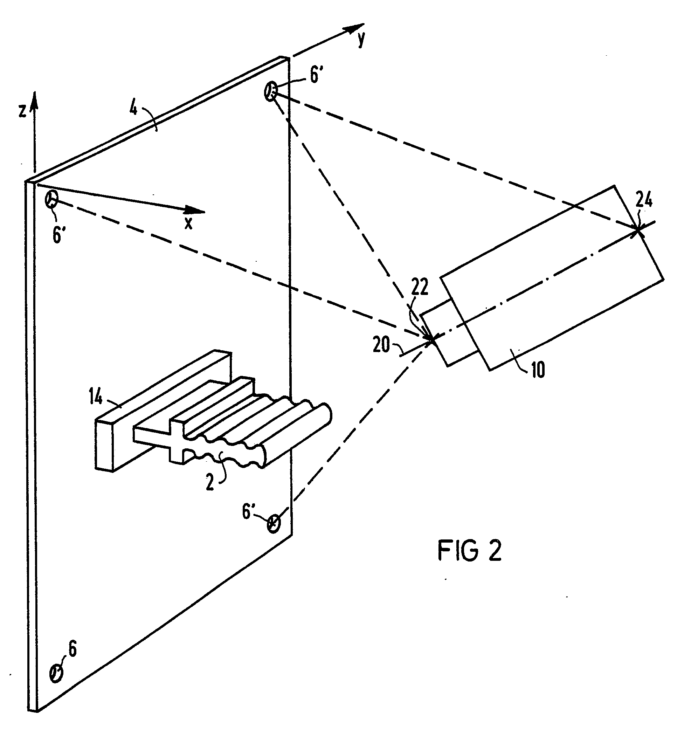

Nach Fig. 1 und 2 wird als Koordinations-Vorrichtung eine Kombination von Beißplatte 2 und starrem Träger 4, der mit Markierungen 6 und 6' versehen ist, verwendet. Diese Kombination wird zur Fixierung des Kopfes 8 eines Probanden oder Patienten gleichermaßen in zwei Untersuchungsgeräten verwendet.

Sie wird verwendet einmal in Verbindung mit einer Meßeinrichtung 10 für biomagnetische Signale und einem medizinischen Schnittbildgerät (nicht gezeigt), beispielsweise sowohl bei einer MEG-Messung in vorgegebener Relation zwischen dem Träger 4 und der MEG-Meßapparatur als auch bei einer MR-Messung im Magnetfeld eines MR-Meßgerätes.

Die Beißplatte 2 ist ein Negativ-Abdruck des Gebisses des Probanden in einer schnell aushärtenden Masse, wie sie beispielsweise beim Zahnarzt verwendet wird. Beißt der Proband auf die Beißplatte 2, ist eine starre Kopplung zwischen Kopf 8 und Beißplatte 2 gewährleistet. Die Beißplatte 2 ist über einen Halter 12 in eine Verbindung 14 an dem Träger 4 einführbar-oder einsteckbar. Diese Verbindung 14 gewährleistet eine lösbare, aber starre Befestigung zwischen Beißplatte 2 und Träger 4. Die Verbindung 14 kann dazu, wie gezeigt, in Form einer randseitig verstärkten Einführöffnung 15 oder eines auf der Innenfläche aufgesetzten Rahmens ausgebildet sein. Die Verbindung 14 kann reproduzierbar einstellbar ausgeführt sein, so daß sie an eine jeweils bequeme Position des Patienten angepaßt werden kann.

Der Träger 4 kann insbesondere als eine ebene Platte aus Kunststoff, die beispielsweise 20 cm mal 20 cm groß ist, ausgeführt sein. In der dargestellten Ausgestaltung besitzt sie noch eine winklig, hier unter 90° angesetzte Seitenplatte 4a, auf der der Kopf 8 des Patienten mit seinem Ohr ruht. Mit dieser Kombination 2, 4 und ggf. 4a kann sowohl eine definierte Positionierung des Kopfes 8 während der Messung biomagnetischer Signale als auch eine Zuordnung der Position der betreffenden Meßeinrichtung 10, z.B. eines entlang eines Doppelpfeiles 16 drehbaren SQUID-Gradiometers, zur aus dem Schnittbild, z.B. aus dem MR-Bild ersichtlichen Kopfgeometrie erreicht werden. Dazu ist der Träger 4 mit Befestigungsmitteln 17, z.B. in Form von Einsteckstiften oder Klammern, versehen, die eine Befestigung am raumfesten Stativ (nicht gezeigt) der Meßeinrichtung 10 ermöglichen.

Bereits bei der Herstellung der Beißplatte 2 ist der länglich ausgestaltete Halter 12 in die Abdruckmasse integriert, so daß eine starre Einheit entsteht. Auf diese Weise kann die Beißplatte 2 über den Halter 12 reproduzierbar, d.h. wiederholt mit hoher Ortsgenauigkeit, mit Hilfe der Verbindung 14 auf die Kunststoff platte 4 aufgesteckt werden. Die Beißplatte 2 wird für jeden Probanden nur einmal hergestellt, was wenig Aufwand bedeutet, und ist dann beliebig oft verwendbar. Der Kopf 8 ist sehr gut fixiert, da die Fixierung über den Kieferknochen und den Schädelknochen starr ist. Die Positionierung zwischen Beißplatte 2 und Kunststoffplatte 4 ist bei festem Biß gut reproduzierbar.

Die Schnittbild-Aufnahme des Kopfes 8, also z.B. die MR-Aufnahme, erfolgt mit Beißplatte 2 und Kunststoff-Platte 4 und ggf. 4a. Eine Fixierung im MR-Gerät ist nicht notwendig. Die Lage des Kopfes 8 relativ zur Kunststoff-Platte 4 kann nachträglich im MR-Bild erfaßt werden, denn die Kunststoff-Platte 4 ist ja mit einer Anzahl Fixpunkten oder Markierungen 6 und 6', z.B. mit wassergefüllten Löchern versehen, die im MR-Bild wiederzufinden sind. Die Reproduzierbarkeit der Positionierung kann sehr genau und einfach mit zwei oder drei MR-Aufnahmen geprüft werden.

Aus Fig. 2 geht hervor, daß bei der Messung biomagnetischer Signale mit Gradiometer (SQUID-Array, Dewar, Halterung, etc.) die Kunststoff-Platte 4 mittels der Mittel problemlos relativ zur Meßapparatur 10 fixiert werden kann. Dann kann die exakte Position, z.B. relativ zum Gradiometer-Array, innerhalb der Meßapparatur 10 gemessen werden. Auch bei einer relativ willkürlichen Befestigung der Platte 4 an der Meßapparatur 10, wie in Fig. 2 angenommen, ist die Bestimmung der Lage des Kopfes 8 relativ zum Gradiometer-Array auf vier einfach und exakt durchzuführende Entfernungsmessungen reduziert. Dies ist in Fig. 2 durch gestrichelte Linien angedeutet. Es muß durch diese Messungen die gegenseitige Lage und Orientierung der Kunststoff-Platte 4 und der Dewarachse 20 der Gradiometer-Meßapparatur 10 bestimmt werden. Wie ersichtlich, genügt dazu die Messung der Entfernung von drei beabstandeten Markierungen 6' auf der Kunststoff-Platte 4 relativ zu einer einzigen Markierung 22 auf dem Dewar und von zwei Markierungen 22, 24 auf dem Dewar relativ zu einer Markierung 6' auf der Kunststoff-Platte 4. Die mathematischen Relationen hierfür ergeben sich aus fundamentalen geometrischen Grundlagen.

In vielen Fällen wird man mit einigen wenigen festen Positionen der Kunststoff-Platte 4 bei der MEG-Messung auskommen. Die Lage der Kunststoff-Platte 4 braucht nur einmal ausgemessen zu werden, wenn die relative Lage des Dewars zum Kopf 8 durch die Bewegung der Dewarhalterung eingestellt werden kann. Dewar-Lage und Dewar-Richtung 20 werden dann an der Dewarhalterung (nicht gezeigt) abgelesen.

Nach Fig. 3 umfaßt eine anders konzipierte Koordinations-Vorrichtung einen Ring 30, der starr um den Kopf 28 eines Patienten geschlungen ist. Eine Verstelleinrichtung 32 dient zur Verstellung des Rings 30 zwecks Anpassung an den Kopfdurchmesser; sie kann wie eine Gürtelschnalle gestaltet sein. An dem Ring 30 befinden sich drei Zeiger 34, 35, 36, von denen nur die Zeiger 34 und 36 direkt sichtbar sind. Diese Zeiger 34, 35, 36 sind jeweils in der Länge veränderbar und jeweils mit einer Skala 44, 45, 46 versehen. Weitere Skalen für die zirkulare Einstellung sind mit 48, 49 bzw. 50 bezeichnet. Die Zeiger 34, 35, 36 werden an drei markante Punkte (Fixpunkte) des Kopfes 28 angelegt, wie z.B. im vorliegenden Beispiel an die Mitte der beiden Ohrkanäle und die Spaltmitte zwischen den Schneidezähnen des Oberkiefers. Alternativ können auch die Nasenspitze 52, das Nasion 54 und/oder das Inion 56 als Fixpunkte verwendet werden. Zwischen den Fixpunkten des Kopfes 28 und den reproduzierbar eingestellten Zeigern 34, 35, 36 besteht somit eine feste Zuordnung. D.h., die Stellung der Zeiger 34, 35, 36 wird notiert und bei den anderen Meßverfahren verwendet.

An dem Ring 30 ist über einen Adapter 60 ein geometrischer Körper 62 angebracht. Dieser Körper 62 ist mit dem Ring 30 über den Adapter 60 fest, d.h. starr verbunden. Er ist für ein vorgegebenes Schnittbild-Diagnose-Verfahren erkennbar geformt, besitzt also z.B. die Form einer Platte, eines Kreuzes oder einer Kreisscheibe, und/oder er ist mit beabstandeten Markierungen 64 versehen. Bei MR-Untersuchungen können die Markierungen 64 wieder flüssigkeitsgefüllte Löcher in geometrisch einfacher Konfiguration, z.B. im Viereck, sein. Die Markierungen 64 definieren Lage und Orientierung des Körpers 62 im Raum, damit auch diejenigen des Bandes 30 und des Kopfes 28. Der starre Ring 30 ist bevorzugt ein nichtmagnetisches Band, das um den Kopf 28 des Patienten geschlungen ist. Der Adapter 60 dient dazu, bei anderen Patienten andere Körper 62 aufzustecken.

Bei Untersuchungen am Thorax des Patienten wird ein entsprechender Ring um den Thorax gelegt. Die an diesem angebrachten Zeiger werden an die Mamillen und an das obere und das untere Ende des Sternums angelegt. Natürlich können auch hier andere Fixpunkte gewählt werden.

Als Diagnoseverfahren zur Herstellung des gewünschten Schnittbildes kommen neben dem obengenannten Verfahren der Kernspin-Tomographie auch die Computer-Tomographie, SPECT, PET und Ultraschall als Bildlieferverfahren für die geometrisch richtige Zuordnung von Signalen in der biomagnetischen Diagnostik in Betracht.

Die in den Figuren 1, 2 und 3 dargestellten Vorrichtungen zur Zuordnung oder Koordination können überall dort zum Einsatz kommen, wo ein Patient mit mehreren Verfahren untersucht wird, insbesondere bei berührungslos arbeitenden Verfahren, und wo bei anschließenden Therapiemaßnahmen oder Operationen eine besonders hohe Genauigkeit bezüglich der Positionierung erforderlich ist.

**Ansprüche**

1. Vorrichtung zur geometrischen Zuordnung von Informationen über einen Untersuchungsgegenstand, die in einem Schnittbildgerät gewonnen werden, zu Meßpunkten, die von demselben Untersuchungsgegenstand in einer Meßeinrichtung für biomagnetische Signale gewonnen werden, **gekennzeichnet durch**

a) eine Beißplatte (2), deren Kontur dem Gebiß des Patienten (8) entspricht, und

b) einen starren Träger (4), der mit der Beißplatte (2) starr verbindbar oder verbunden und der mit Markierungen (6, 6') versehen ist.

2. Vorrichtung nach Anspruch 1, **dadurch gekennzeichnet**, daß der Träger (4) für die Markierungen (6, 6') eine Platte ist.

3. Vorrichtung nach Anspruch 2, **dadurch gekennzeichnet**, daß die Platte aus Kunststoff besteht.

4. Vorrichtung nach Anspruch 2 oder 3, **dadurch gekennzeichnet**, daß die Platte eben ist.

5. Vorrichtung nach Anspruch 2, 3 oder 4, **dadurch gekennzeichnet**, daß die Platte etwa 20 cm mal 20 cm groß ist.

6. Vorrichtung nach einem der Ansprüche 1 bis 5, **dadurch gekennzeichnet**, daß die Beißplatte (2) aus einem schnell aushärtenden Kunststoff besteht.

7. Vorrichtung nach einem der Ansprüche 1 bis 6, **dadurch gekennzeichnet**, daß die Markierungen (6, 6') solche sind, die in einem MR-Bild sichtbar sind.

8. Vorrichtung nach einem der Ansprüche 1 bis 7, **dadurch gekennzeichnet**, daß die Markierungen wassergefüllte Höhlungen im Träger (4) sind.

9. Vorrichtung nach einem der Ansprüche 1 bis 8, **dadurch gekennzeichnet**, daß der Träger (4) mit drei beabstandeten Markierungen (6') versehen ist.

10. Vorrichtung nach einem der Ansprüche 1 bis 9, **dadurch gekennzeichnet**, daß der Träger (4) mit Befestigungsmitteln (17) versehen ist, die eine Befestigung an einer Meßeinrichtung (10) für biomagnetische Signale ermöglichen.

11. Vorrichtung nach einem der Ansprüche 1 bis 10, **dadurch gekennzeichnet**, daß der Träger (4) über eine reproduzierbar einstellbare Verbindung (14) mit der Beißplatte (2) verbindbar ist.

12. Vorrichtung nach einem der Ansprüche 1 bis 11, **gekennzeichnet durch**
einen Halter (12), der an der Beißplatte (2) befestigt und der mit dem Träger (4) verbindbar oder verbunden ist.

13. Vorrichtung zur geometrischen Zuordnung von Informationen über einen Untersuchungsgegenstand, die in einem Schnittbildgerät gewonnen werden, zu Meßpunkten, die von demselben Untersuchungsgegenstand in einer Meßeinrichtung für biomagnetische Signale gewonnen werden, **gekennzeichnet durch**

a) einen starren Ring (30), der um den Patienten (28) schlingbar ist,

b) eine Anzahl in der Länge veränderbarer Zeiger (34, 35, 36), die jeweils mit einer Skala (44, 45, 46) versehen und am Ring (30) angebracht sind, und

c) einen mit dem Ring (30) verbundenen Körper (62), der für ein vorgegebenes Diagnoseverfahren erkennbar geformt oder markiert ist derart, daß seine Lage und Orientierung im Raum definiert sind.

14. Vorrichtung nach Anspruch 13, **dadurch gekennzeichnet**, daß der Körper (62) eine eckige Platte, ein Kreuz oder eine Kreisscheibe ist.

15. Vorrichtung nach Anspruch 13 oder 14, **dadurch gekennzeichnet**, daß der Körper (62) mit Markierungen (64) versehen ist.

16. Vorrichtung nach Anspruch 15, **dadurch gekennzeichnet**, daß die Markierungen (64) wassergefüllte Höhlungen im besagten Körper (62) sind.

17. Vorrichtung nach einem der Ansprüche 13 bis 16, **dadurch gekennzeichnet**, daß der starre Ring (30) ein nichtmagnetisches Band ist, das um den Kopf (28) des Patienten schlingbar ist.

18. Vorrichtung nach einem der Ansprüche 13 bis 17, **dadurch gekennzeichnet**, daß drei Zeiger (34, 35, 36) vorgesehen sind.

19. Vorrichtung nach einem der Ansprüche 13 bis 18, **dadurch gekennzeichnet**, daß die Länge eines (36) der Zeiger (34, 35, 36) dem Abstand zwischen Stirn und Schneidezähnen des Patienten (28) entspricht.

20. Vorrichtung nach einem der Ansprüche 13 bis 19, **dadurch gekennzeichnet**, daß die Länge zweier Zeiger (34, 35) dem Abstand zwischen Kopfhaut und Ohrkanal des Patienten (28) entspricht.

21. Vorrichtung nach einem der Ansprüche 13 bis 20, **dadurch gekennzeichnet**, daß der Körper (62) mit dem Ring (30) über einen Adapter (60) verbunden ist.

FIG 1

FIG 2

FIG 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 193 650 (H. DELNON) <br> * Zusammenfassung; Seite 5, Zeilen 1-6; Seite 6, Zeilen 22-30; Seite 14, Zeilen 12-25; Seite 17, Zeilen 8-16; Seite 19, Zeilen 20-33; Seite 21, Zeilen 4-20; Seite 24, Zeilen 17-20; Seite 26, Zeilen 8-11,20-28; Figuren 1,2,4A-4C * | 1,11 | A 61 B 6/08 <br> A 61 B 5/05 <br> A 61 B 5/04 |
| A | --- | 6,12 | |
| Y | IEEE TRANSACTIONS ON BIOMEDICAL ENGINEERING, Band BME-32, Nr. 6, Juni 1985, Seiten 455-458, IEEE, New York, US; W.J.J. KOUIJZER et al.: "Neuromagnetic fields evoked by a patterned on-offset stimulus" <br> * Seiten 455-456, Zusammenfassung; Abschnitt II: "Method"; Figur 1 * | 1,11 | |
| A | Idem <br> --- | 3,4 | |
| A | US-A-3 577 160 (J.E. WHITE) <br> * Zusammenfassung; Spalte 1, Zeilen 10-18; Spalte 3, Zeilen 3-33; Spalte 4, Zeilen 1-35,51-75; Figuren 1,8-11,15 * <br> --- | 2-4,9, 13-15, 17 | RECHERCHIERTE SACHGEBIETE (Int. Cl.4) <br><br> A 61 B |
| A | EP-A-0 018 276 (COMPAGNIE GENERALE DE RADIOLOGIE) <br> * Zusammenfassung; Seite 4, Zeile 31 - Seite 5, Zeile 15; Seite 6, Zeilen 15-27; Seite 8, Zeilen 1-18; Seite 16, Zeilen 8-32; Figuren 1-7B * <br> ---      -/- | 13-15, 17 | |

Der vorliegende Recherchenbericht wurde für alle Patentansprüche erstellt

| Recherchenort | Abschlußdatum der Recherche | Prüfer |
|---|---|---|
| DEN HAAG | 28-10-1988 | RIEB K.D. |

EPO FORM 1503 03.82 (P0403)

Europäisches Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-4 319 136 (J.R. JINKINS)<br>* Zusammenfassung; Spalte 2, Zeilen 1-33; Spalte 3, Zeile 10 - Spalte 4, Zeile 53; Spalte 5, Zeilen 6-20; Figuren 1-5 *<br>--- | 13,15 | |
| A | DE-A- 517 356 (A. RONA)<br>* Seite 1, Zeile 48 - Seite 2, Zeile 75; Figuren 1-4 *<br>----- | 13,15, 18 | |

RECHERCHIERTE SACHGEBIETE (Int. Cl.4)

Der vorliegende Recherchenbericht wurde für alle Patentansprüche erstellt

| Recherchenort | Abschlußdatum der Recherche | Prüfer |
|---|---|---|
| DEN HAAG | 28-10-1988 | RIEB K.D. |

EPO FORM 1503 03.82 (P0403)